# EUROPEAN PATENT SPECIFICATION

(11) **EP 3 761 935 B1**
(45) Date of publication and mention of the grant of the patent: **27.11.2024**
(21) Application number: 19712089.2
(22) Date of filing: 05.03.2019
(51) Int. Cl.: A61F 13/62, A61F 13/551, A61F 13/56

(54) **ADHESIVE TAPE ASSEMBLIES**
KLEBEBANDANORDNUNGEN
ENSEMBLES BANDES ADHÉSIVES

(30) Priority: 05.03.2018 US 201862638383 P
(43) Date of publication of application: 13.01.2021
(73) Proprietor: Avery Dennison Corporation, Mentor, OH 44060 (US)
(72) Inventor: BOGAERTS, Bert, 2530 Boechout (BE)
(74) Representative: HGF
(86) International application number: PCT/US2019/020696
(87) International publication number: WO 2019/173297

(56) References cited:
- EP-A1- 1 214 034
- WO-A1-2017/180702
- WO-A2-2012/158538
- US-A1- 2004 249 357
- US-A1- 2014 088 543

## Description

### CROSS-REFERENCE TO RELATED APPLICATION

The present application claims the benefit of U.S. Provisional Application No. 62/638,383, filed March 5, 2018.

### BACKGROUND

Fastening tabs may be found in connection with disposable absorbent articles such as diapers as well as other applications. Disposable absorbent articles, such as baby diapers and/or incontinence diapers for adults, are generally known in the art. A disposable article (for example, a diaper chassis), in many instances, is constructed of a liquid absorbent core (promoting separation of fluid from the user) enclosed between a liquid permeable topsheet (located adjacent to a wearer when the disposable article is worn) and a liquid impermeable backsheet (forming an outer surface of the disposable article when worn, which may be a non-woven fabric laminated with a water impermeable layer such as a polyethylene film). The disposable article generally includes a rear portion intended to cover a wear's behind, a front portion intended to cover a wear's front and a crotch portion there between.

Disposable articles are available in reusable form which must be washed between uses, or may be one-time disposable forms. Disposable forms include one or more adhesive tape assemblies which are typically used to maintain a used diaper in a "rolled-up" form after use to facilitate handling and disposal.

Although currently known tape assemblies used in disposable articles are satisfactory in many respects, in many instances during disposal one or more regions of adhesive in the fastener tape assemblies are exposed and can inadvertently "stick" to waste containers or the user attempting disposal. Further, the tape assemblies may contain a significant amount of adhesive used due to the length of these fastener tape assemblies.

In addition, disposable absorbent articles are also provided with one or more other forms of fastening tabs. The fastening tabs may be in the form of a tape. The tabs typically have a face coated with an adhesive. The adhesive may be a pressure sensitive adhesive. The fastener tape assembly generally includes adhesive tabs fastened to one end of the disposable article assembly construction at each lateral side of the disposable article in a permanent "factory joint" by the disposable article or diaper manufacturer using adhesives or other techniques. For example, two fastening tabs may be joined to the rear portion of the disposable article along opposing side ends or edges thereof. The fastening tabs may be joined directly to the chassis, or alternately, via intervening side panels. The fastening tabs allow each side of the rear portion of the disposable article to be releasably attached in a nonpermanent "user joint" (to allow unfastening to inspect the disposable article) to the front portion of the disposable article thereby selectively forming a waistband around the wearer.

In many applications, the front portion of the disposable article is provided with a landing strip (also referred to herein as a "landing zone") arranged thereon to selectively receive the fastening tabs. For example, this landing strip may include a female portion of a mechanical fastening system. In particular, the landing strip may include loop material designed to be engaged by corresponding hooks carried on the fastening tabs. The landing zone may provide a plastic surface of a non-woven surface and may comprise a knit type fabric landing pad.

Securing a fastener to a disposable article at the factory joint is typically performed so as to provide a strong and durable affixment. Several techniques have been used including mechanical bonding procedures and the use of high strength adhesives. In order to reduce the cost of manufacturing disposable articles, certain portions of the fastening tabs may contain patterns of adhesive. However, there can be difficulties in developing a fastening system that can achieve a desirable degree of peel adhesion and/or shear strength when the fastening tabs with adhesive are attached directly to the disposable article backsheet. This problem is particularly evident in baby diapers which generally have smaller fastening tabs (due to their relatively smaller size) as compared to adult incontinence diapers which can generally accommodate larger fastening tabs. Further, increasing the length of the fastening tabs may also provide increased costs related to the increased use of adhesive to cover the fastening tabs needed to secure the disposable article or substrate in its intended use. A means to reduce the amount of adhesive used on fastener tape assemblies yet providing a similar or increased length of the fastener tape assemblies would be desirable.

WO2017180702A discloses various fastening tape closure assemblies. The closure tapes are useful in the manufacture of disposable articles, and particularly disposable diapers. The closure tapes generally include a fastening component, an extension component, and a bonding component. The tape systems provide a new folding configuration and reduced adhesive along a face of the extension component. Also described are disposable articles utilizing the tape closure assemblies.

### SUMMARY

In accordance with a first aspect of the present invention there is provided a fastener tape assembly comprising a fastening component defining a first face for affixment to a disposable article or substrate and an oppositely directed second face; a connecting component, wherein the connecting component comprises a bonding component defining a first face for affixment to a disposable article or substrate and an oppositely directed second face; and an extension component generally disposed between the fastening component and the bonding component; wherein the first face of the fastening component is at least partially covered by an adhesive; wherein the first face of the bonding component is at least partially covered by an adhesive and wherein the extension component comprises a folded edge and further comprises an adhesive-free portion between the folded edge of the extension component and the bonding component. The folded edge of the extension component may be generally disposed between the fastening component and the bonding component. In many embodiments, the extension component is essentially free of adhesive. In many other embodiments, the extension component is at least partially covered by an adhesive. In some embodiments, the extension component is at least partially covered by an adhesive wherein the adhesive of the extension component is at least partially patterned. In some embodiments, the extension component is at least partially covered by an adhesive wherein the adhesive of the extension component is a pressure sensitive adhesive.

As used herein, diaper may also be referred to as an article or a disposable article. The diaper may be an adult diaper or a baby diaper. The diaper described herein may also be a pant diaper.

As used herein, essentially free of adhesive means that any adhesives have not been intentionally added to the extension component.

The fastener tape assembly comprises a bonding component defining a first face for affixment to a disposable article or substrate and an oppositely directed second face in which the first face of the bonding component is at least partially covered by an adhesive to thereby bond the bonding component to a disposable article or substrate. In some embodiments, the bonding component is separately formed or manufactured separately from the extension component, and the bonding component has an adhesive region on the first face of the bonding component which is at least partially covered by an adhesive to thereby at least partially bond the bonding component with the extension component. As used herein, at least partially bonded means that the bond is such that it can secure the portion of the fastener tape assembly to either another portion of the fastener tape assembly or a disposable article or substrate, but may not fully bond to the entire portion to which it is adhered, being either another portion of the fastener tape assembly or a disposable article or substrate. At least partially bonded may also mean that it is at least partially affixed or adhered to the assembly.

In other embodiments, the bonding component and extension component are manufactured together as one piece or unit and are attached to one another during manufacturing. After manufacturing, the adhesive of the bonding component is applied to the first face of the bonding component.

In many embodiments, the adhesive on the first face of the bonding component is at least partially covered by an adhesive. In some embodiments, the adhesive on the first face of the bonding component is at least partially patterned. In some embodiments, the adhesive of the bonding component is a pressure sensitive adhesive.

The fastener tape assembly also comprises a fastening component in which an adhesive of the fastening component is provided in an adhesive region on the first face of the fastening component to thereby bond the fastening component with the extension component and/or a disposable article or substrate. In many embodiments, the first face of the fastening component is at least partially covered by an adhesive to thereby bond the fastening component with the extension component and/or a disposable article or substrate. In some embodiments, the first face of the fastening component further comprises hooks.

In many embodiments, the adhesive on the first face of the fastening component is at least partially covered by an adhesive. In some embodiments, the adhesive on the first face of the fastening component is at least partially patterned. In some embodiments, the adhesive of the fastening component is a pressure sensitive adhesive.

The fastener tape assembly additionally comprises an extension component generally disposed between the fastening component and the bonding component. In many embodiments, the folded edge of the extension component may be generally disposed between the fastening component and the bonding component. In many embodiments, the folded edge of the extension component may be a U-bond or a Y-bond. In many embodiments, the extension component is essentially free of adhesive. In many embodiments, the extension component is at least partially covered by an adhesive. In some embodiments, the extension component is at least partially covered by an adhesive wherein the adhesive of the extension component is at least partially patterned. In some embodiments, the extension component is at least partially covered by an adhesive wherein the adhesive of the extension component is a pressure sensitive adhesive. In other embodiments, the extension component may be at least partially attached, bonded, or affixed to the bonding component by some other means of attachment, which may include but is not limited to, melt bonding, ultrasonic bonding, and separate attachment tapes.

In some embodiments, the fastener tape assembly further comprises a finger lift at least partially affixed to the fastening component. In many embodiments, the fastener tape assembly further comprises a release layer on at least one of the bonding component and the extension component. In some embodiments, fastener tape assembly further comprises at least one perforation in the fastening component. In many embodiments, the fastening component and connecting component of the fastener tape assembly may be of varying lengths for each of the components. Additionally, the fastening component, the extension component, and the bonding component of the fastener tape assembly may be of varying lengths for each of the components.

In many embodiments, the fastener tape assembly is in a roll form.

The present subject matter provides a method of making the fastener tape assembly described herein. The present subject matter provides an article including the fastener tape assembly described herein. In many embodiments, the article includes the fastener tape assembly described herein. In many embodiments, the article is a disposable article. In many embodiments, the article is a diaper. In other embodiments, the diaper is a pant diaper. The literal scope of the present invention is set out in the appended claims.

In many embodiments, the article may include a fastener tape assembly. In many embodiments, the fastener tape assembly comprises: 1) a fastening component defining a first face for affixment to a disposable article or substrate and an oppositely directed second face; 2) and a connecting component. The connecting component further comprises a) a bonding component defining a first face for affixment to a disposable article or substrate and an oppositely directed second face; and b) an extension component generally disposed between the fastening component and the bonding component. The first face of the fastening component is at least partially covered by an adhesive; and the first face of the bonding component is at least partially covered by an adhesive; and the extension component comprises a folded edge and further comprises an adhesive-free portion between the folded edge of the extension component and the bonding component. In many embodiments, the folded edge of the extension component may be generally disposed between the fastening component and the bonding component.. In many embodiments, the extension component is essentially free of adhesive. In many other embodiments, the extension component is at least partially covered by an adhesive. In some embodiments, the extension component is at least partially covered by an adhesive wherein the adhesive of the extension component is at least partially patterned. In some embodiments, the extension component is at least partially covered by an adhesive wherein the adhesive of the extension component is a pressure sensitive adhesive.

As will be realized, the subject matter described herein is capable of other and different embodiments and its several details are capable of modifications in various respects, all without departing from the claimed subject matter. Accordingly, the drawings and description are to be regarded as illustrative and not restrictive.

### BRIEF DESCRIPTION OF THE DRAWINGS

FIGURE 1A is a schematic illustration showing a disposable article including a fastening tab according to aspects described herein.
FIGURE 1B is a schematic illustration showing a disposable article including a fastening tab according to aspects described herein
FIGURE 2 is a schematic illustration of a fastener tape assembly in accordance with aspects described herein.
FIGURE 3 is a schematic illustration of another fastener tape assembly in accordance with aspects described herein.
FIGURE 4 is a schematic illustration of another fastener tape assembly in accordance with aspects described herein.
FIGURE 5 is a schematic illustration of another fastener tape assembly in accordance with aspects described herein.
FIGURE 6 is a schematic illustration of another fastener tape assembly in accordance with aspects described herein.
FIGURE 7 is a schematic illustration of another fastener tape assembly in accordance with aspects described herein.
FIGURE 8 is a schematic illustration of another fastener tape assembly in accordance with aspects described herein.
FIGURE 9 is a schematic illustration of another fastener tape assembly in accordance with aspects described herein.
FIGURE 10 is a schematic illustration of another fastener tape assembly in accordance with aspects described herein.
FIGURE 11 is a schematic illustration of another fastener tape assembly in accordance with aspects described herein.
FIGURE 12 is a schematic illustration of another fastener tape assembly in accordance with aspects described herein
FIGURE 13 is a schematic illustration of another fastener tape assembly in accordance with aspects described herein
FIGURE 14 is a schematic illustration of another fastener tape assembly in accordance with aspects described herein.
FIGURE 15 is a schematic illustration of another fastener tape assembly in accordance with aspects described herein.
FIGURE 16 is a schematic illustration of another fastener tape assembly in accordance with aspects described herein.
FIGURE 17 is a schematic illustration of another fastener tape assembly in accordance with aspects described herein.
FIGURE 18 is a schematic illustration of another fastener tape assembly in accordance with aspects described herein.

### DETAILED DESCRIPTION OF THE EMBODIMENTS

The present disclosure relates generally to the fastener tape assemblies and articles that may utilize these fastener tape assemblies. These fastener tape assemblies may be used in disposal of certain articles. The present subject matter may provide similar or increased lengths for the fastener tape assemblies. The present subject matter may also provide a reduction in the amount of adhesives typically used for fastener tape assemblies. The present subject matter may also provide a reduction in costs associated with a reduction in the amount of adhesives typically used for fastener tape assemblies.

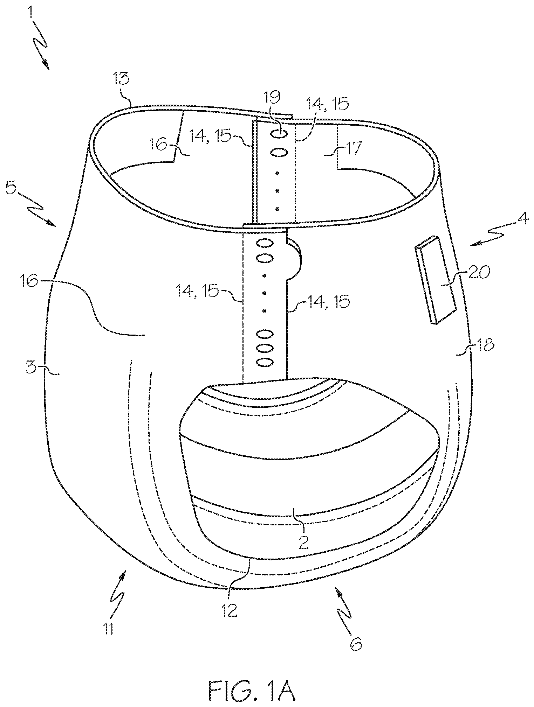

FIGURE 1A illustrates an embodiment of a disposable article 1 in accordance with the present subject matter. Referring to FIGURE 1A, the disposable article 1 has a front portion 5, a rear portion 4, and a crotch portion 6 between the front portion 5 and the rear portion 4. A chassis 11 is provided in the front portion 5, rear portion 4, and crotch portion 6. The chassis 11 may include a topsheet 2 which may be liquid pervious, a backsheet 3 which may be liquid impervious and is associated with the topsheet 2, and an absorbent core (not shown) disposed between the topsheet 2 and the backsheet 3.

In many embodiments, the backsheet 3 may be suitably formed from an appropriate filmic material, for example nonwoven material or other materials such as, but not limited to, polyethylene. In some embodiments, the backsheet 3 is formed from a nonwoven material having a weight in the range of approximately 7 gsm to approximately 25 gsm (grams per square meter), or in the range of approximately 10 gsm to approximately 20 gsm, or in the range of approximately 12 gsm to approximately 18 gsm. In some embodiments, the nonwoven material suitably may include a spunbond material. In some embodiments, the nonwoven material suitably may include an entangled or otherwise arranged collection of fibers or filaments that may be thermally bonded or adhesively bonded to a polymer web or backing (e.g., a polyethylene and/or polypropylene backing). Of course, alternately, other known nonwoven materials may be similarly used and/or other known methods for manufacturing the nonwoven material may be employed.

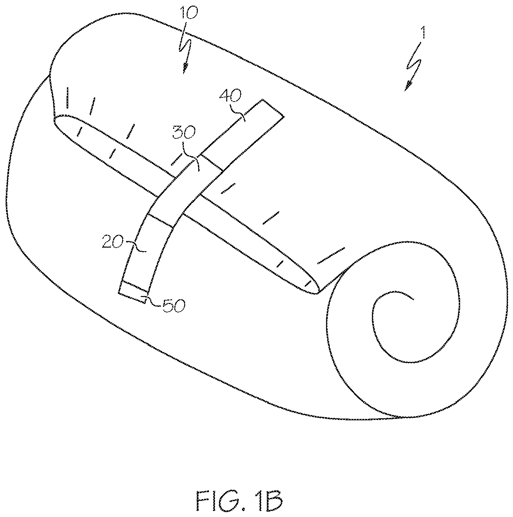

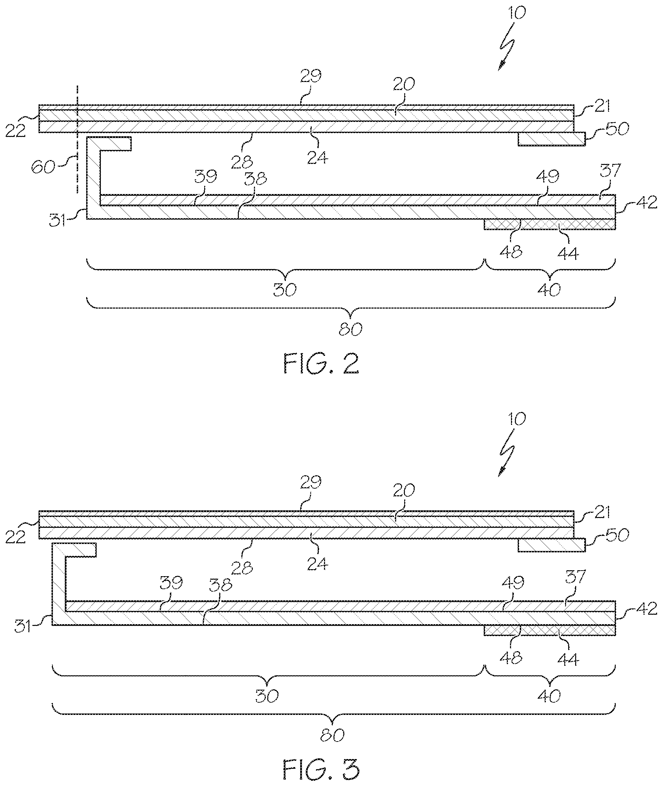

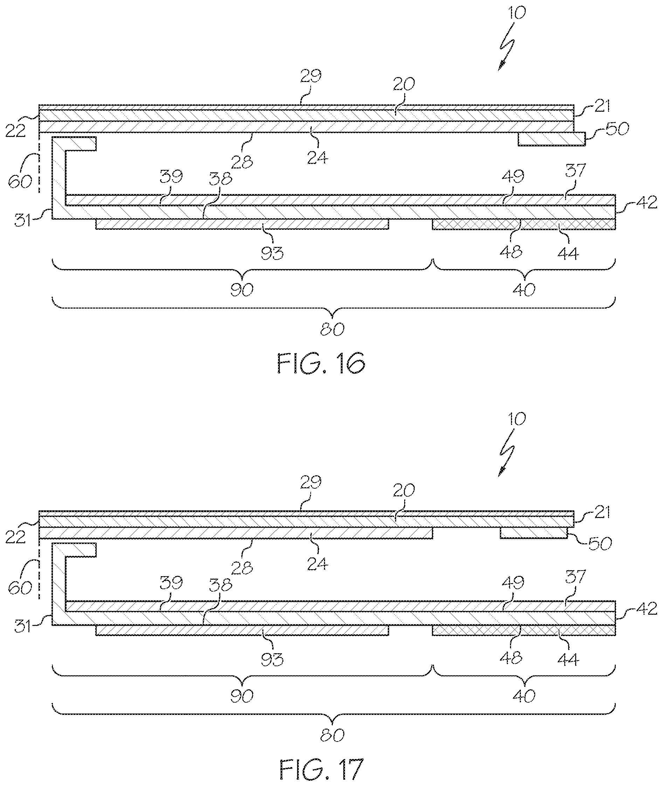

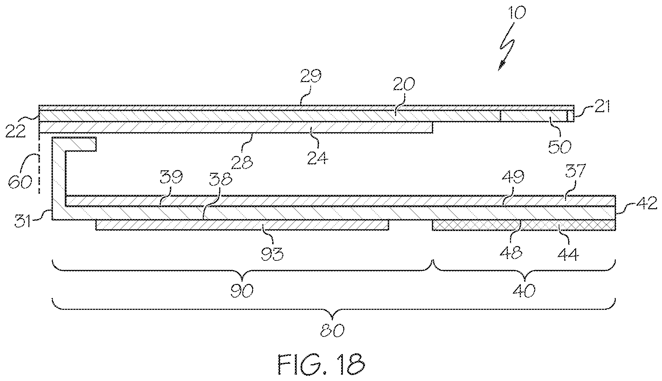

The disposable article 1 may further include a pair of front ear panels (16 and 17), each extending laterally outward from the corresponding sides of the chassis 11 in the front portion 5, and a pair of extensible back ear panels 16, 17 each extending laterally outward from the corresponding sides of the chassis 11 in the rear portion 4. Each of the ear panels 16, 17 has an outermost edge 14 which forms an outermost edge line 15. The disposable article 1 may further include seams 19 each joining the front and back ear panels 16, 17 along the corresponding outermost edge lines 15 to form the two leg openings 12 and the waist opening 13. The disposable article 1 includes a fastener tape assembly 10 (shown in FIGURE 1B) where the fastening component 20 may be viewed on the edge 18 of the disposable article 1 in some embodiments. Although fastener tape assembly 10 is described herein, it will be understood that the subject matter includes other tape assemblies, such as the fastener tape assemblies described in FIGURES 2-18.

After the disposable article 1 has been soiled, the soiled disposable article 1 is torn open along the seams 19 to remove the soiled disposable article 1 from the wearer. Alternatively, if appropriate, the soiled disposable article 1 may be removed from the wearer by pulling down without tearing open the seams 19. The disposable article 1 may then be folded or rolled up by keeping the crotch portion 6 in the center so that the fastener assembly 10 is exposed along the outside of the rolled disposable article 1 to facilitate handling for a convenient disposal as shown in FIGURE 1B, while containing the contents within the rolled disposable article 1. FIGURE 1B also illustrates a fastener tape assembly 10 of a disposable article 1, including a finger lift 50 provided proximate the distal edge 21 of the fastening component 20, a bonding component 40, and an extension component 30 generally disposed between the fastening component 20 and the bonding component 40.

FIGURES 2-18 illustrate various features and aspects of the present subject matter of fastener tape assemblies 10 of the present subject matter. The fastener tape assembly 10 described herein comprises: 1) a fastening component 20 defining a first face 28 for affixment to a disposable article 1 or substrate and an oppositely directed second face 29; 2) and a connecting component 80. The connecting component 80 further comprises: a) a bonding component 40 defining a first face 48 for affixment to a disposable article or substrate and an oppositely directed second face 49; and b) an extension component (shown as either 30 or 90, described further below) generally disposed between the fastening component 20 and the bonding component 40.

As described herein, the fastening component 20 comprises both a connecting edge 22 and a distal edge 21. The connecting edge 22 of the fastening component 20 is at least partially affixed or bonded to the extension component 30. In many embodiments, the connecting edge 22 of the fastening component 20 extends beyond the extension component 30. The fastening component 20 comprises a first face 28 and a second face 29, and adhesive may be applied in the adhesive region 24. In many embodiments, the first face 28 of the fastening component 20 is at least partially covered by an adhesive to thereby bond the fastening component 20 with the extension component (shown as either 30 or 90) and/or a disposable article or substrate. As further described herein, the adhesive region 24 may comprise adhesive that is continuous or patterned (not shown in the figures). As used herein, a patterned adhesive may describe an adhesive layer that includes adhesive-free regions in a particular pattern. In some embodiments, the adhesive on the first face 28 of the fastening component 20 is at least partially patterned. In some embodiments, the adhesive on the first face 28 of the fastening component 20 is a pressure sensitive adhesive.

In many embodiments in FIGURES 2-18, the first face 28 of the fastening component 20 is at least partially covered by an adhesive. The adhesive on the first face 28 of the fastening component 20 may allow the fastening component 20 to adhere or bond with the extension component (shown as either 30 or 90).

As described herein in FIGURES 2-18, the bonding component 40 comprises a corresponding edge 42. The corresponding edge 42 of the bonding component 40 describes the outer edge of the bonding component 40. In many embodiments, the first face 48 of the bonding component 40 is at least partially covered by an adhesive in the adhesive region 44 to thereby bond the bonding component 40 with the extension component (shown as either 30 or 90) and/or a disposable article or substrate. The adhesive on the first face 48 of the bonding component 40 may allow the bonding component 40 to adhere or bond with the extension component (shown as either 30 or 90). In some embodiments, the adhesive on the first face 48 of the bonding component 40 may be a pressure sensitive adhesive. In some embodiments, the adhesive on the first face 48 of the bonding component 40 may be a patterned adhesive. In some embodiments, the adhesive on the first face 48 of the bonding component 40 is at least partially patterned. In some embodiments, the adhesive of the bonding component 40 is a pressure sensitive adhesive.

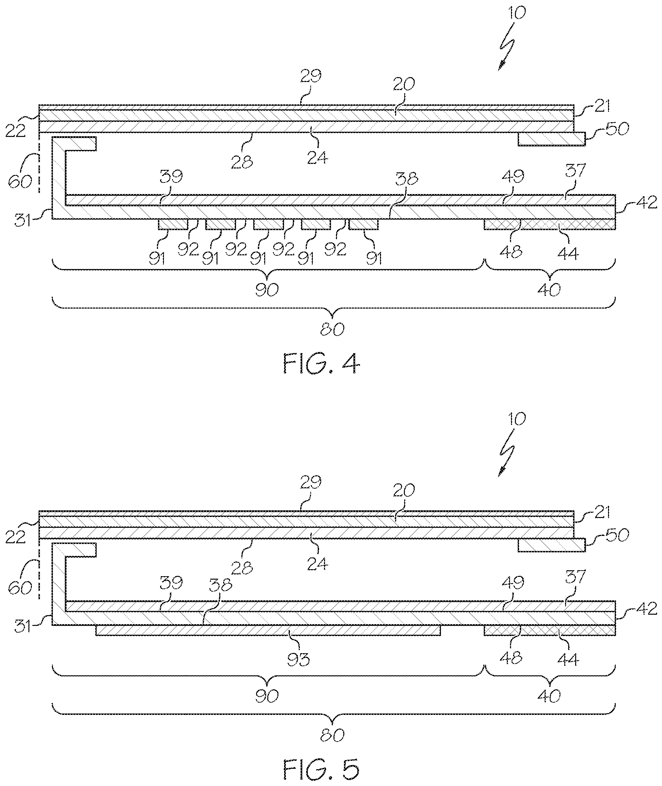

Further, in many embodiments in FIGURES 2-18, namely FIGURES 2, 3, and 6-14, the extension component 30 is essentially free of adhesive. While both the fastening component 20 and bonding component 40 have adhesive applied to these components, the extension component 30 does not contain any adhesive, but is at least partially attached, affixed, or bonded to both the fastening component 20 and bonding component 40 by the adhesives on these components at their adhesive regions (24 and 44, respectively). In many other embodiments in FIGURES 2-18, namely FIGURES 4, 5, and 15-18, the extension component 90 (unlike extension component 30) is at least partially covered by an adhesive 91. In some embodiments, the extension component is at least partially covered by an adhesive 91 wherein the adhesive of the extension component is at least partially patterned, as shown by the areas with no adhesive 92. In some embodiments, the extension component is at least partially covered by an adhesive wherein the adhesive of the extension component is a pressure sensitive adhesive.

In many embodiments in FIGURES 2-18, the adhesives described above and used for the fastening component 20, bonding component 40, and/or extension component 90 (shown only in FIGURES 4, 5, and 15-18) may be rubber-based adhesives. In some embodiments, the rubber adhesive is comprised of styrene-isoprene-styrene (SIS), styrene-butadiene-styrene (SBS), styrene-butadiene-styrene (SBS)/styrene-butadiene (SB), or combinations thereof. Copolymers and/or derivatives of these and other adhesives could be utilized. The referenced figures illustrate typical regions of adhesive within the fastener tape assemblies 10. In other embodiments, the adhesives may be acrylic adhesives, silicone adhesives, polyurethane adhesives, or hybrid adhesives.

Additionally, adhesive coat weights for all of the noted adhesive regions in the fastening component 20 and the bonding component 40 (24 and 44, respectively) typically are from about 20 g/m² (known as gsm, or grams per square meter) to about 50 g/m² (gsm). However, the present subject matter may include coat weights less than or greater than these values.

In many embodiments, the fastener tape assembly 10 additionally comprises an extension component (shown as either 30 or 90) generally disposed between the fastening component 20 and the bonding component 40. The extension component (shown as either 30 or 90) defines an opposing face 39 oppositely directed from the adhesive-free face 38. In many embodiments, the extension component (shown as either 30 or 90) may have a folded edge 31 that may be generally disposed between the fastening component 20 and the bonding component 40.

In some embodiments, fastener tape assembly 10 further comprises at least one optional perforation 60 in the fastening component 20, as shown in FIGURE 2. The at least one optional perforation 60 may allow a user to tear at least a portion of the fastener tape assembly 10 within the fastening component 20 for ease of removal of the fastener tape assembly 10 from the disposable article or other article. The optional perforation 60 shown in FIGURE 2 provides not only at least a partial bonding of the tape but also access to a convenient tape opening that can provide a release from the diaper on that part of the tape in order to either free up the extendability or provide a breakage point. In some embodiments, the optional perforation 60 in FIGURE 2 may be a part of a longer fastener tape assembly 10 than one without the optional perforation 60. In many embodiments, the connecting edge 22 of the fastening component 20 extends beyond the extension component 30. In other embodiments, the portion of the connecting edge 22 of the fastening component 20 may serve to at least partially bond the edge of the fastener tape assembly 10. If the connecting edge 22 is used for bonding, then it may allow for: 1) ease of processing the fastener assembly tape 10: 2) ease in peeling the fastener tape assembly 10 from the disposable article 1: and/or 3) decreased delamination of the disposable article 1.

In many embodiments, the folded edge 31 of the extension component (shown as either 30 or 90) may be in a variety of different forms including, but not limited to, a U-bond or a Y-bond in order to facilitate attachment to the fastening component 20. The folded edge 31 is positioned to partially extend over to the adhesive region 24 of the first face 28 of the fastening component 20. Use of the folded edge 31 provides a secure means to at least partially bond the extension component (shown as either 30 or 90) and the fastening component 20. Further, the folded edge 31 also aids in the extension of the fastener tape assembly 10. Additionally, but not shown in the figures, the extension component (shown as either 30 or 90) may comprise bonding strip which may at least partially bond the extension component (shown as either 30 or 90) to the fastening component 20. In other embodiments, the extension component (shown as either 30 or 90) may not have a folded edge 31, but another means of attachment to the fastening component 20 (not shown in the figures). In other embodiments, the extension component (shown as either 30 or 90) may be at least partially attached, bonded, or affixed to the fastening component 20 by some other means of attachment, which may include but is not limited to, melt bonding, ultrasonic bonding, and separate attachment tapes.

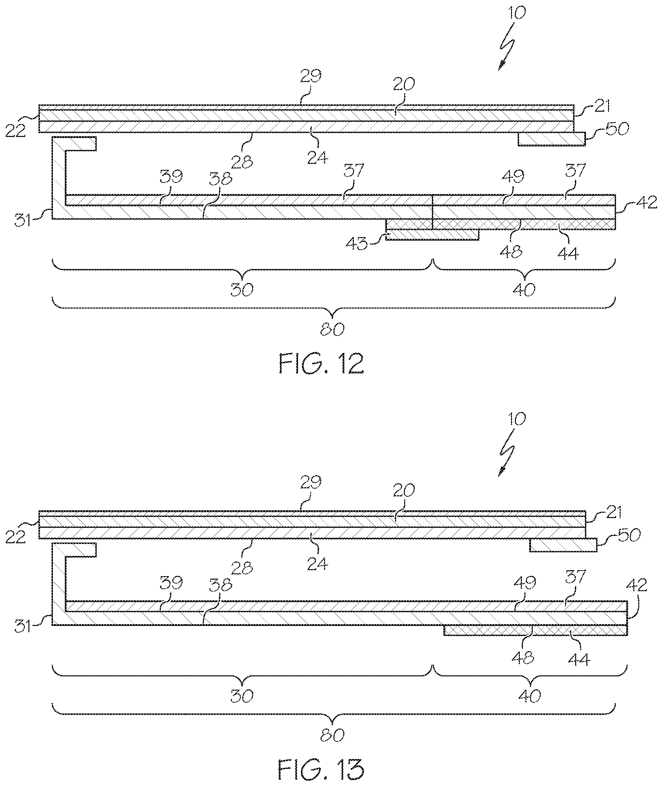

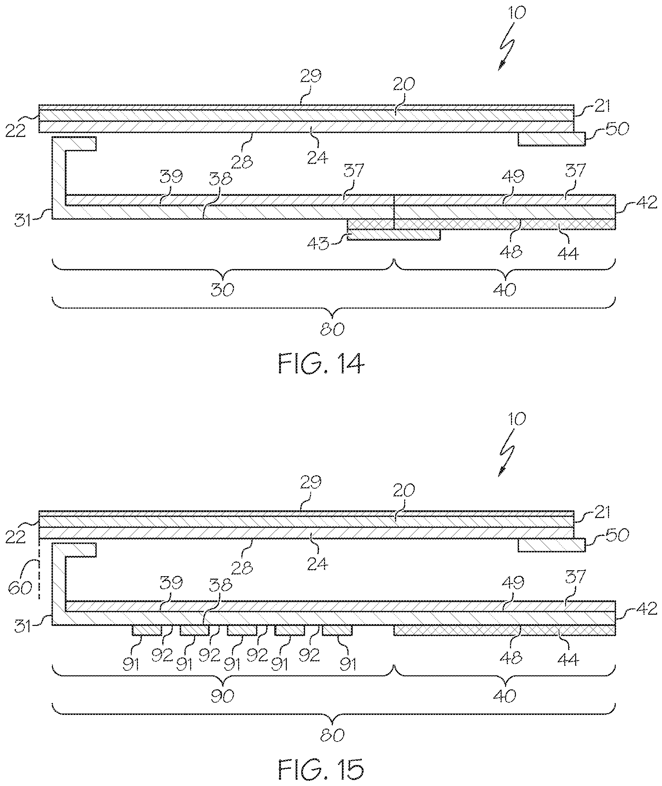

In many embodiments, the connecting component 80 may be manufactured in different ways to form the fastener tape assembly 10. In some embodiments (as shown in FIGURES 2, 3, 9, and 11-14), the bonding component 40 and extension component (shown as either 30 or 90) are originally manufactured together as one piece or unit, which is referred to collectively as the connecting component 80. Subsequently, the adhesive of the bonding component 40 is applied to the first face 48 of the bonding component 40 in the adhesive region 44. In many embodiments, the adhesive of the bonding component 40 is subsequently applied to the first face 48 of the bonding component 40 in the adhesive region 44 after the bonding component 40 and extension component 30 are manufactured together as one piece or unit. Alternatively, the bonding component 40 and extension component (shown as either 30 or 90) may be manufactured separately and at least partially connected to the fastening component 20. In many embodiments, the adhesive of the bonding component 40 is provided in an adhesive region on the first face 48 of the bonding component 40 to thereby at least partially bond the bonding component 40 with the extension component (shown as either 30 or 90). In some embodiments, the bonding component 40 and extension component (shown as either 30 or 90) may be manufactured separately and at least partially bonded together by a connecting strip 43. FIGURES 12 and 14 provide a connecting strip 43 to aid in connecting the connecting component 30 and the bonding component 40.

In many embodiments, at least one of the bonding component 40 and extension component (shown as either 30 or 90) may have a release layer 37 (as shown in FIGURES 2-18) at least partially disposed on the opposing face 39. In some embodiments, the release layer 37 may be at least partially disposed onto the bonding component 40 in a process separate from the release layer 37 partially disposed on the extension component (30 or 90). In other embodiments, the release layer 37 may be at least partially disposed onto the bonding component 40 and the extension component (30 or 90) in one process. In many embodiments, the release layer 37 may be a siliconized material. The release layer 37 may serve to allow the tape to open due to its release properties. In some embodiments, the folded edge 31 of the extension component (30 or 90) may provide at least partial bonding in combination with the adhesive region 44 of the corresponding edge 42 of the bonding component 40. In yet other embodiments, another release coating or treatment may be used for the release layer 37 rather than or in addition to a siliconized material.

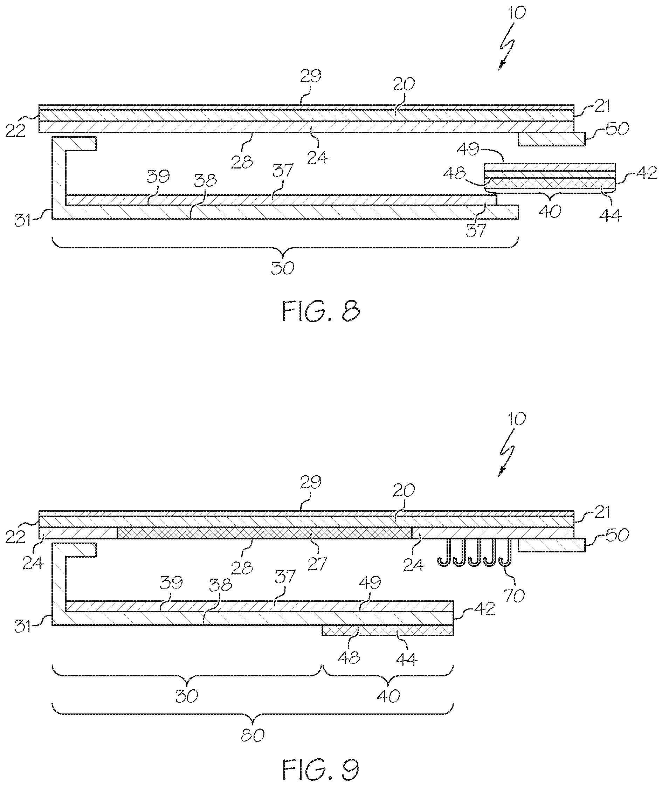

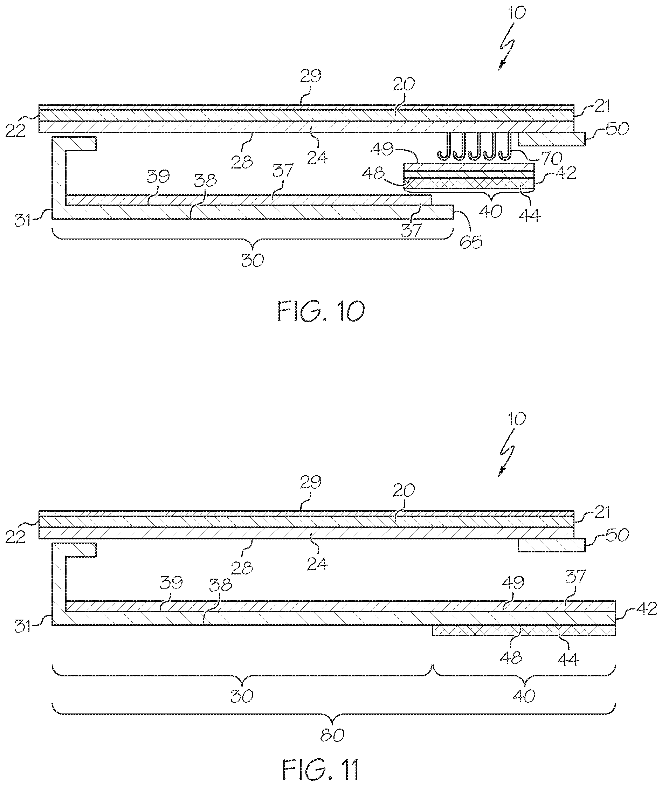

In some embodiments (as shown in FIGURES 8 and 10), the bonding component 40 is separately formed or manufactured separately from the extension component (shown as either 30 or 90), and the bonding component 40 has a region on the first face 48 of the bonding component 40 which is at least partially covered by an adhesive to thereby at least partially bond the bonding component 40 with the extension component (shown as either 30 or 90). The bonding component 40 and the extension component (shown as either 30 or 90) are then at least partially bonded together to form the connecting component 80. In some embodiments, the adhesive in the adhesive region 44 on the first face 48 of the bonding component 40 may be a patterned adhesive. As used herein, at least partially bonded means that the bond is such that it can secure the portion of the fastener tape assembly 10 to either another portion of the fastener tape assembly 10 or a disposable article 1 or substrate, but may not fully bond to the entire portion to which it is adhered, being either another portion of the fastener tape assembly 10 or a disposable article 1 or substrate. At least partially bonded may also mean that it is at least partially affixed or adhered to the fastener tape assembly 10.

A variety of polymeric films and materials can be used for one or more of the fastening component 20, extension component (shown as either 30 or 90), and/or bonding component 40. Nonlimiting examples of polymeric films include polypropylene and/or polyethylene. Nonwoven materials and nonwoven laminates can also be used in the fastener tape assemblies 10. In many embodiments, the extension component (shown as either 30 or 90) may comprise the folded edge 31 (in many embodiments, U-bonds or Y-bonds), which can be formed from appropriately configured polypropylene films and layer(s) of nonwoven materials. In other embodiments, the fastening component 20, extension component (shown as either 30 or 90), and/or bonding component 40 may comprise elastic components for increased stretchability. In yet other embodiments, the fastening component 20, extension component (shown as either 30 or 90), and/or bonding component 40 may be made of stiffer materials for improved bonding to the disposable article 1. In some embodiments, the fastening component 20, extension component (shown as either 30 or 90), and/or bonding component 40 may have similar properties with respect to stretchability and stiffness. In other embodiments, the fastening component 20, extension component (shown as either 30 or 90), and/or bonding component 40 may have different properties with respect to stretchability and stiffness.

In many embodiments, the fastening component 20 has a thickness within a range of from about 30 µm to about 120 µm and particularly from about 50 µm to about 90 µm. The extension component (shown as either 30 or 90) has a thickness within a range of from about 30 µm to about 120 µm, and particularly from about 50 µm to about 90 µm. The bonding component 40 has a thickness within a range of from about 15 µm to about 120 µm, and particularly from about 20 µm to about 50 µm. The total thickness of the fastener tape assembly 10 is typically within a range of from about 100 µm to about 600 µm, and in many embodiments from about 250 µm to about 500 µm.

In some embodiments, the fastener tape assembly further comprises a finger lift 50 at least partially affixed to the fastening component 20. In some versions of the fastener tape assemblies 10, a finger lift 50 is provided proximate the distal edge 21 of the fastening component 20. The finger lift 50 may be at least partially attached to the first face 28 of the fastening component 20 by an adhesive in an adhesive region 24. In some embodiments, the adhesive in the adhesive region 24 may be a patterned adhesive. In some embodiments, the finger lift 50 typically includes a tab (not shown) for gripping by a user and a region of adhesive (not shown) for securing the tab to the fastening component 20 and typically to the first face 28.

In some embodiments, the first face of the fastening component further comprises hooks 70. The hooks 70 described herein are provided in FIGURES 9 and 10. In particular, the landing strip of the disposable article 1 may include loop material designed to be engaged by corresponding hooks carried on the fastening tabs. In many embodiments, the landing strip may be a nonwoven backsheet or outer belt to anchor the hooks. The landing zone may provide a plastic surface of a non-woven surface and may comprise a knit type fabric landing pad. Alternatively, a loop may be provided in the bonding component 40 for anchoring purposes.

Referring now to FIGURE 2, the fastener tape assembly 10 provided comprises The fastener tape assembly 10 described herein comprises: 1) a fastening component 20 defining a first face 28 for affixment to a disposable article 1 or substrate and an oppositely directed second face 29; and 2) and a connecting component. The connecting component further comprises a) a bonding component 40 defining a first face 48 for affixment to a disposable article 1 or substrate and an oppositely directed second face 49; and b) an extension component 30 generally disposed between the fastening component 20 and the bonding component 40. In FIGURE 2, the fastening component 20 comprises both a connecting edge 22 and a distal edge 21. The connecting edge 22 of the fastening component 20 is at least partially affixed or bonded to the extension component 30. The fastening component 20 comprises a first face 28 and a second face 29, and adhesive may be applied in the adhesive region 24. In many embodiments, the first face 28 of the fastening component 20 is at least partially covered by an adhesive to thereby bond the fastening component 20 with the extension component 30 and/or a disposable article 1 or substrate. Further in FIGURE 2, both the optional perforation 60 and finger lift (described above) 50 are both provided. Additionally, the bonding component 40 and extension component 30 (collectively together, the connecting component 80) are manufactured together as one piece and the adhesive of the bonding component 40 is applied to the first face 48 of the bonding component 40 in FIGURE 2.

The present subject matter also provides variations of the fastener tape assembly 10 depicted in FIGURE 2. FIGURES 3 and 5-18 illustrate a number of other embodiments of fastener tape assembly 10 provided in FIGURES 2 and 4 where FIGURE 2 provides an extension component 30 with no adhesive and FIGURE 4 provides an extension component 90 which is at least partially covered by an adhesive. In these figures, the fastener tape assemblies 10 comprise the same or similar components as previously described in association with the fastener tape assembly 10 of FIGURES 2 and 4. However, the fastener tape assemblies shown in FIGURES 3 and 5-18 include several variations as compared to the fastener tape assembly 10 of FIGURES 2 and 4 as follows.

The fastener tape assembly 10 shown in FIGURE 3 corresponds to the previously described fastener tape assembly 10 of FIGURE 2 except for that at least one optional perforation 60 is only provided in FIGURE 2. FIGURE 3 has no optional perforations.

The fastener tape assembly 10 shown in FIGURE 5 corresponds to the previously described fastener tape assembly 10 of FIGURE 4 except for that the extension component 90 in FIGURE 5 is at least partially covered by an adhesive 93 and the adhesive has no patterning.

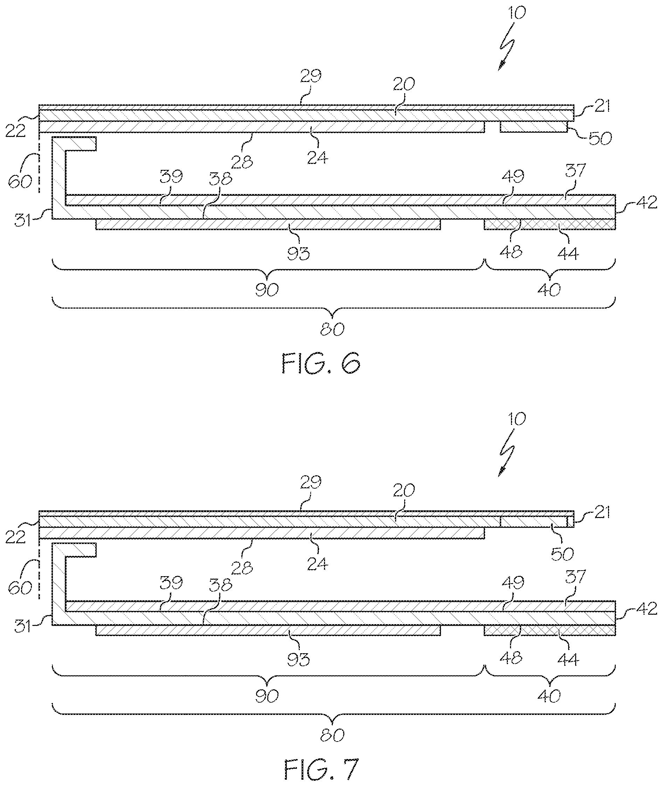

The fastener tape assembly 10 shown in FIGURE 6 corresponds to the previously described fastener tape assembly 10 of FIGURE 5 except for that the adhesive region 24 of the fastening component 20 is only partially covered with adhesive.

The fastener tape assembly 10 shown in FIGURE 7 corresponds to the previously described fastener tape assembly 10 of FIGURE 6 except that the finger lift 50 may be printed rather than added as a separate attached film. The printing may be done on either the inside or outside of the fastener tape assembly 10.

In FIGURE 8, the bonding component 40 and extension component 30 are manufactured separately and the adhesive of the bonding component 40 is provided in an adhesive region 44 on the first face 48 of the bonding component 40 to thereby at least partially bond the bonding component 40 with the extension component 30. Once the bonding component 40 and extension component 30 are at least partially bonded to each other, the connecting component 80 is then formed. Unlike FIGURE 4, the bonding component 40 and extension component 30 are manufactured together as one piece or and the adhesive of the bonding component 40 is applied to the first face 48 of the bonding component 40 in FIGURE 2. In some embodiments, there may be overlap with the bonding component and the release layer 37.

FIGURE 9 corresponds to the previously described fastener tape assembly 10 of FIGURE 2 except for that there is a series of hooks 70 on the first face 28 of the fastening component 20 that are at least partially attached or bonded to the fastening component by adhesive in the adhesive region 24. Further, the adhesive region, unlike FIGURE 2, is not continuous in FIGURE 9. Instead, there is at least one adhesive free region 27 in FIGURE 9.

FIGURE 10 corresponds to the previously described fastener tape assembly 10 of FIGURE 9 except for that the bonding component 40 and extension component 30 are manufactured separately and the adhesive of the bonding component 40 is provided in an adhesive region 44 on the first face 48 of the bonding component 40 to thereby at least partially bond the bonding component 40 with the extension component 30 in FIGURE 10. Unlike FIGURE 10, the bonding component 40 and extension component 30 are manufactured together as one piece or and the adhesive of the bonding component 40 is applied to the first face 48 of the bonding component 40 in FIGURE 9.

FIGURES 11-18 provide some example dimensions (in nm) for the parts of the fastener tape assembly 10. FIGURES 11-18 may show some of the features described herein and illustrated in FIGURES 1-10. Referring further to the fastener tape assembly 10 described herein and in many embodiments of the present matter, the dimension for folded edge 31 (also referred to as the "foldover") is within a range of from about 4 mm to about 10 mm, and more particularly from about 4 mm to about 6 mm, with 5 mm shown in FIGURE 11-18. A typical total fastener tape assembly width shown as "W" is from about 50 mm to about 80 mm, with total widths of about 70.3 mm, about 69 mm, about 66 mm (see FIGURES 13 and 14), and about 62 mm (see FIGURES 11 and 12) being used for certain applications. Dimensions shown in FIGURES 11-18 may be described below in Table 1.

**Table 1: Tape Dimensions (in mm)**

| | FIG. 11 | FIG. 12 | FIG. 13 | FIG. 14 | FIG. 15 | FIG. 16 | FIG. 17 | FIG. 18 |
|---|---|---|---|---|---|---|---|---|
| Fastening Component 20 | 57.5 | 57.5 | 61.5 | 61.5 | 61.5 | 61.5 | 61.5 | 61.5 |
| Extension Component 30 | 40 | 40 | 44 | 39 | 39 | 44 | 44 | 44 |
| Bonding Component 40 | 20 | 20 | 20 | 25 | 25 | 20 | 20 | 20 |
| Difference of Connecting Component Fastening Component (80-20) | 3.5 | 3.5 | 3.5 | 3.5 | 3.5 | 3.5 | 3.5 | 3.5 |
| Finger lift 50 overlap | | | 0.78 | 0.78 | 0.78 | 0.78 | 0.78 | 0.78 |
| Portion of Bonding Component 40 on Fastening Component 20 | 5 | 5 | 5 | 5 | 5 | 5 | 5 | 5 |
| Length of Connecting Strip 43 (if present) | | 10 | | 10 | | | | |

Additionally, the fastening component 20, the extension component 30, and the bonding component 40 of the fastener tape assembly 10 may be of varying lengths for each of the components. Further, the fastening component 20 and the connecting component 80 of the fastener tape assembly 10 may be of varying lengths for each of the components. In some embodiments, the distal edge 21 the fastening component 20 extend beyond the length that includes the extension component (shown as either 30 or 90) and corresponding edge 42 of the bonding component 40. In other embodiments, the distal edge 21 of the fastening component 20 extends beyond the separate extension edge of the extension component (shown as either 30 or 90) where the extension component (shown as either 30 or 90) is manufactured separately from the bonding component 40. In some embodiments, the length that includes the extension component (shown as either 30 or 90) and corresponding edge 42 of the bonding component 40 (collectively, the connecting component 80) may extend beyond the distal edge 21 the fastening component 20. In yet other embodiments, the length that includes the extension component (shown as either 30 or 90) and corresponding edge 42 of the bonding component 40 (collectively, the connecting component 80) may be about the same length as the distal edge 21 the fastening component 20.

As noted, use of the fastener tape assembly 10 described herein can, in certain applications, provide an increased length of the fastening tabs, which may also provide the decreased use of adhesive to cover the fastening tabs needed to secure the disposable article 1 or substrate in its intended use. A means to reduce the amount of adhesive used on fastener tape assemblies yet allowing a similar or increased length of the fastener tape assemblies would be desirable. In certain instances, the fastener tape assembly 10 means to reduce the amount of adhesive used on fastener tape assemblies yet allowing a similar or increased length of the fastener tape assemblies 10 would be desirable. A decrease in the amount of adhesive used in a fastener tape assembly 10 may also provide decreased costs in manufacturing the fastener tape assembly. In many embodiments, the fastener tape assembly is in a roll form.

The present subject matter provides a method of making the fastener tape assembly described herein.

The present subject matter also includes a wide array of articles that include the noted fastener tape assemblies described herein. In many embodiments, the articles are disposable, i.e., the articles are formed from materials that can be recycled and/or which are relatively inexpensive. Nonlimiting examples of such articles include diapers, training pants, adult diapers, and in particular pant diapers.

In another aspect, the present subject matter provides an article including a fastener tape assembly. In many embodiments, the article is a disposable article. In many embodiments, the article is a diaper or another substrate. The diaper may be a baby diaper or an adult diaper. In other embodiments, the diaper is a pant diaper.

In many embodiments, the fastener tape assembly included in the article comprises: 1) a fastening component defining a first face for affixment to a disposable article or substrate and an oppositely directed second face; and 2) a connecting component. The connecting component further comprises a) a bonding component defining a first face for affixment to a disposable article or substrate and an oppositely directed second face; and b) an extension component generally disposed between the fastening component and the bonding component. In many embodiments, the first face of the fastening component is at least partially covered by an adhesive. In some embodiments, the adhesive on the first face of the fastening component may be a pressure sensitive adhesive. In some embodiments, the adhesive on the first face of the fastening component may be a patterned adhesive. In many embodiments, the first face of the bonding component is at least partially covered by an adhesive. In some embodiments, the adhesive on the first face of the bonding component may be a pressure sensitive adhesive. In some embodiments, the adhesive on the first face of the bonding component may be a patterned adhesive. In many embodiments, the extension component is essentially free of adhesive. In many other embodiments, the extension component is at least partially covered by an adhesive. In some embodiments, the extension component is at least partially covered by an adhesive wherein the adhesive of the extension component is at least partially patterned. In some embodiments, the extension component is at least partially covered by an adhesive wherein the adhesive of the extension component is a pressure sensitive adhesive. For the fastener tape assembly included in the article, the article may be a diaper. In many embodiments, the diaper maybe a pant diaper. In some embodiments for the article, the bonding component and extension component may be manufactured separately and the adhesive of the bonding component is provided in an adhesive region on the first face of the bonding component to thereby at least partially bond the bonding component with the extension component. In other embodiments for the article, the bonding component and extension component are manufactured together as one piece or and the adhesive of the bonding component is applied to the first face of the bonding component

Many other configurations of the fastener tape assemblies and articles with the fastener tape assemblies are contemplated. Some are now discussed herein, but may not be provided in the Figures. The present subject matter includes all operable combinations of features and aspects described herein. Thus, for example if one feature is described in association with an embodiment and another feature is described in association with another embodiment, it will be understood that the present subject matter includes embodiments having a combination of these features. While the invention has been described in detail, modifications within the spirit and scope of the invention will be readily apparent to those of skill in the art.

In addition, it should be understood that aspects of the invention and portions of various embodiments and various features recited below and/or in the appended claims may be combined or interchanged either in whole or in part. In the foregoing descriptions of the various embodiments, those embodiments which refer to another embodiment may be appropriately combined with other embodiments as will be appreciated by one of skill in the art without departing from the principle and scope of the claimed subject matter, as expressed in the appended claims. Furthermore, those of ordinary skill in the art will appreciate that the foregoing description is by way of example only, and is not intended to limit the invention.

## Claims

1. A fastener tape assembly (10) comprising:
a fastening component (20) defining a first face (28) for affixment to a disposable article (1) or substrate and an oppositely directed second face (29);
a connecting component (80), wherein the connecting component (80) comprises:
a bonding component (40) defining a first face (48) for affixment to a disposable article (1) or substrate and an oppositely directed second face (49); and
an extension component (30, 90) generally disposed between the fastening component (20) and the bonding component (40);
wherein the first face (28) of the fastening component (20) is at least partially covered by an adhesive;
wherein the first face (48) of the bonding component (40) is at least partially covered by an adhesive; and
wherein the extension component (30, 90) comprises a folded edge (31) and further comprises an adhesive-free portion (27) between the folded edge (31) of the extension component (30, 90) and the bonding component (40).

2. The fastener tape assembly (10) of Claim 1 wherein the adhesive of the fastening component (20) is provided in an adhesive region on the first face (28) of the fastening component (20) to thereby bond the fastening component (20) with the extension component (30, 90), optionally wherein the fastening component (20) further comprises hooks (70), optionally wherein the adhesive of the fastening component (20) is at least partially patterned, optionally wherein the adhesive of the fastening component (20) is a pressure sensitive adhesive.

3. The fastener tape assembly (10) of Claim 1 or claim 2, wherein the bonding component (40) and extension component (30, 90) are manufactured separately and the adhesive of the bonding component (40) is provided in an adhesive region (44) on the first face (48) of the bonding component (40) to thereby at least partially bond the bonding component (40) with the extension component (30, 90); or
wherein the bonding component (40) and extension component (30, 90) are manufactured together as one piece or unit and the adhesive of the bonding component (40) is applied to the first face (48) of the bonding component (40).

4. The fastener tape assembly (10) of any one of Claims 1-3 wherein the adhesive of the bonding component (40) is at least partially patterned, optionally wherein the adhesive of the bonding component (40) is a pressure sensitive adhesive, optionally wherein the folded edge (31) of the extension component (30, 90) is a U-bond or a Y-bond.

5. The fastener tape assembly (10) of any one of Claims 1-4 wherein the fastener assembly (10) further comprises a finger lift (50) at least partially affixed to the fastening component (20), optionally wherein the fastener assembly (10) further comprises at least one perforation (60) in the fastening component (20), optionally wherein a connecting edge (22) of the fastening component (20) extends beyond the extension component (30, 90).

6. The fastener tape assembly (10) of any one of Claims 1-5 wherein the extension component (30, 90) and the bonding component (40) extend beyond the distal edge (21) of the fastening component (20), optionally wherein the distal edge (21) of the fastening component (20) extends beyond the extension component (30, 90) and the bonding component (40), optionally wherein the distal edge (21) of the fastening component (20) extends beyond the separate extension edge of the extension component (30, 90).

7. The fastener tape assembly (10) of any one of Claims 1-6 wherein the extension component (30, 90) is essentially free of adhesive; or
wherein the extension component (30, 90) is at least partially covered by an adhesive, optionally wherein the adhesive of the extension component (30, 90) is at least partially patterned, optionally wherein the adhesive of the extension component (30, 90) is a pressure sensitive adhesive.

8. The fastener tape assembly (10) of any one of Claims 1-7 further comprising a release layer (37) on at least one of the bonding component (40) and the extension component (30, 90), optionally further comprising at least one optional perforation (60) in the fastening component (20).

9. The fastener tape assembly (10) of any one of Claims 1-8 wherein the fastener tape assembly (10) is in a roll form.

10. The method of making the fastener tape assembly (10) of any of Claims 1-9, the method comprising the steps of:
providing a fastening component (20) defining a first face (28) for affixment to a disposable article (1) and an oppositely directed second face (29);
providing a connecting component (80) comprising a bonding component (40) defining a first face (48) for affixment to a disposable article or substrate and an oppositely directed second face (49); and
providing an extension component (30, 90) disposed between the fastening component (20) and the bonding component (40),
at least partially covering the first face (28) of the fastening component (20) with an adhesive;
at least partially covering the first face (48) of the bonding component (40) with an adhesive; and
providing the extension component (30, 90) with a folded edge (31) and an adhesive free portion between the folded edge (31) and the bonding component (40).

11. An article including a fastener tape assembly (10) of any of Claims 1-9.

12. An article (1) including a fastener tape assembly (10), the fastener tape assembly (10) comprising:
a fastening component (20) defining a first face (28) for affixment to a disposable article (1) or substrate and an oppositely directed second face (29); and
a connecting component (80), wherein the connecting component (80) comprises:
a bonding component (40) defining a first face (48) for affixment to a disposable article (1) or substrate and an oppositely directed second face (49); and
an extension component (30, 90) generally disposed between the fastening component (20) and the bonding component (40);
wherein the first face (28) of the fastening component (20) is at least partially covered by an adhesive;
wherein the first face (48) of the bonding component (40) is at least partially covered by an adhesive; and
wherein the extension component (30, 90) comprises a folded edge (31) and further comprises an adhesive-free portion (27) between the folded edge (31) of the extension component (30, 90) and the bonding component (40).

13. The article (1) of Claim 12, wherein the fastener tape assembly (10) comprises the extension component (30, 90) essentially free of adhesive; or
wherein the fastener tape assembly (10) comprises the extension component (30, 90) at least partially covered by an adhesive, optionally wherein the adhesive of the extension component (30, 90) is at least partially patterned, optionally wherein the adhesive of the extension component (30, 90) is a pressure sensitive adhesive.

14. The article (1) of Claim 12 wherein the article (1) is a diaper, optionally wherein the diaper is a pant diaper.

15. The article (1) of any one of Claims 12-14 wherein the bonding component (40) and extension component (30, 90) are manufactured separately and the adhesive of the bonding component (40) is provided in an adhesive region on the first face (48) of the bonding component (40) to thereby at least partially bond the bonding component (40) with the extension component (30, 90); or
wherein the bonding component (40) and extension component (30, 90) are manufactured together as one piece or unit and the adhesive of the bonding component (40) is applied to the first face (48) of the bonding component (40).

## Patentansprüche

1. Befestigungsbandanordnung (10), umfassend:
eine Befestigungskomponente (20), die eine erste Fläche (28) zur Anbringung an einem Einwegartikel (1) oder Substrat und eine entgegengesetzt gerichtete zweite Fläche (29) definiert;
eine Verbindungskomponente (80), wobei die Verbindungskomponente (80) Folgendes umfasst:
eine Haftungskomponente (40), die eine erste Fläche (48) zur Anbringung an einem Einwegartikel (1) oder Substrat und eine entgegengesetzt gerichtete zweite Fläche (49) definiert; und
eine Verlängerungskomponente (30, 90), die im Allgemeinen zwischen der Befestigungskomponente (20) und der Haftungskomponente (40) angeordnet ist;
wobei die erste Fläche (28) der Befestigungskomponente (20) mindestens teilweise durch einen Kleber bedeckt ist;
wobei die erste Fläche (48) der Haftungskomponente (40) mindestens teilweise durch einen Kleber bedeckt ist; und
wobei die Verlängerungskomponente (30, 90) eine gefaltete Kante (31) umfasst und ferner einen kleberfreien Abschnitt (27) zwischen der gefalteten Kante (31) der Verlängerungskomponente (30, 90) und der Haftungskomponente (40) umfasst.

2. Befestigungsbandanordnung (10) nach Anspruch 1, wobei der Kleber der Befestigungskomponente (20) in einem Klebebereich auf der ersten Fläche (28) der Befestigungskomponente (20) bereitgestellt ist, um dadurch eine Haftung zwischen der Befestigungskomponente (20) und der Verlängerungskomponente (30, 90) herzustellen, wobei die Befestigungskomponente (20) optional ferner Haken (70) umfasst, wobei der Kleber der Befestigungskomponente (20) optional mindestens teilweise strukturiert ist, wobei der Kleber der Befestigungskomponente (20) optional ein druckempfindlicher Kleber ist.

3. Befestigungsbandanordnung (10) nach Anspruch 1 oder Anspruch 2, wobei die Haftungskomponente (40) und die Verlängerungskomponente (30, 90) getrennt gefertigt sind und der Kleber der Haftungskomponente (40) in einem Klebebereich (44) auf der ersten Fläche (48) der Haftungskomponente (40) bereitgestellt ist, um dadurch eine mindestens teilweise Haftung zwischen der Haftungskomponente (40) und der Verlängerungskomponente (30, 90) herzustellen; oder
wobei die Haftungskomponente (40) und die Verlängerungskomponente (30, 90) zusammen als ein Teil oder eine Einheit gefertigt sind und der Kleber der Haftungskomponente (40) auf die erste Fläche (48) der Haftungskomponente (40) aufgetragen ist.

4. Befestigungsbandanordnung (10) nach einem der Ansprüche 1-3, wobei der Kleber der Haftungskomponente (40) mindestens teilweise strukturiert ist, wobei der Kleber der Haftungskomponente (40) optional ein druckempfindlicher Kleber ist, wobei optional die gefaltete Kante (31) der Verlängerungskomponente (30, 90) eine U-Haftungsstelle oder eine Y-Haftungsstelle ist.

5. Befestigungsbandanordnung (10) nach einem der Ansprüche 1-4, wobei die Befestigungsanordnung (10) ferner einen Anhebestreifen (50) umfasst, der mindestens teilweise an der Befestigungskomponente (20) angebracht ist, wobei die Befestigungsanordnung (10) optional ferner mindestens eine Perforation (60) in der Befestigungskomponente (20) umfasst, wobei sich optional eine Verbindungskante (22) der Befestigungskomponente (20) über die Verlängerungskomponente (30, 90) hinaus erstreckt.

6. Befestigungsbandanordnung (10) nach einem der Ansprüche 1-5, wobei sich die Verlängerungskomponente (30, 90) und die Haftungskomponente (40) über die distale Kante (21) der Befestigungskomponente (20) hinaus erstrecken, wobei sich optional die distale Kante (21) der Befestigungskomponente (20) über die Verlängerungskomponente (30, 90) und die Haftungskomponente (40) hinaus erstreckt, wobei sich optional die distale Kante (21) der Befestigungskomponente (20) über die separate Verlängerungskante der Verlängerungskomponente (30, 90) hinaus erstreckt.

7. Befestigungsbandanordnung (10) nach einem der Ansprüche 1-6, wobei die Verlängerungskomponente (30, 90) im Wesentlichen frei von Kleber ist; oder
wobei die Verlängerungskomponente (30, 90) mindestens teilweise durch einen Kleber bedeckt ist, wobei optional der Kleber der Verlängerungskomponente (30, 90) mindestens teilweise strukturiert ist, wobei optional der Kleber der Verlängerungskomponente (30, 90) ein druckempfindlicher Kleber ist.

8. Befestigungsbandanordnung (10) nach einem der Ansprüche 1-7, ferner eine Ablöseschicht (37) auf mindestens einer von der Haftungskomponente (40) und der Verlängerungskomponente (30, 90) umfassend, optional ferner mindestens eine optionale Perforation (60) in der Befestigungskomponente (20) umfassend.

9. Befestigungsbandanordnung (10) nach einem der Ansprüche 1-8, wobei die Befestigungsbandanordnung (10) in einer Rollenform vorliegt.

10. Verfahren zum Herstellen der Befestigungsbandanordnung (10) nach einem der Ansprüche 1-9, wobei das Verfahren die folgenden Schritte umfasst:
Bereitstellen einer Befestigungskomponente (20), die eine erste Fläche (28) zur Anbringung an einem Einwegartikel (1) und eine entgegengesetzt gerichtete zweite Fläche (29) definiert;
Bereitstellen einer Verbindungskomponente (80), die eine Haftungskomponente (40) umfasst, die eine erste Fläche (48) zur Anbringung an einem Einwegartikel oder Substrat und eine entgegengesetzt gerichtete zweite Fläche (49) definiert; und
Bereitstellen einer Verlängerungskomponente (30, 90), die zwischen der Befestigungskomponente (20) und der Haftungskomponente (40) angeordnet ist,
mindestens teilweises Bedecken der ersten Fläche (28) der Befestigungskomponente (20) mit einem Kleber;
mindestens teilweises Bedecken der ersten Fläche (48) der Haftungskomponente (40) mit einem Kleber; und
Bereitstellen der Verlängerungskomponente (30, 90) mit einer gefalteten Kante (31) und einem kleberfreien Abschnitt zwischen der gefalteten Kante (31) und der Haftungskomponente (40).

11. Artikel, der eine Befestigungsbandanordnung (10) nach einem der Ansprüche 1-9 enthält.

12. Artikel (1), der eine Befestigungsbandanordnung (10) enthält, wobei die Befestigungsbandanordnung (10) Folgendes umfasst:
eine Befestigungskomponente (20), die eine erste Fläche (28) zur Anbringung an einem Einwegartikel (1) oder Substrat und eine entgegengesetzt gerichtete zweite Fläche (29) definiert; und
eine Verbindungskomponente (80), wobei die Verbindungskomponente (80) Folgendes umfasst:
eine Haftungskomponente (40), die eine erste Fläche (48) zur Anbringung an einem Einwegartikel (1) oder Substrat und eine entgegengesetzt gerichtete zweite Fläche (49) definiert; und
eine Verlängerungskomponente (30, 90), die im Allgemeinen zwischen der Befestigungskomponente (20) und der Haftungskomponente (40) angeordnet ist;
wobei die erste Fläche (28) der Befestigungskomponente (20) mindestens teilweise durch einen Kleber bedeckt ist;
wobei die erste Fläche (48) der Haftungskomponente (40) mindestens teilweise durch einen Kleber bedeckt ist; und
wobei die Verlängerungskomponente (30, 90) eine gefaltete Kante (31) umfasst und ferner einen kleberfreien Abschnitt (27) zwischen der gefalteten Kante (31) der Verlängerungskomponente (30, 90) und der Haftungskomponente (40) umfasst.

13. Artikel (1) nach Anspruch 12, wobei die Befestigungsbandanordnung (10) die Verlängerungskomponente (30, 90) umfasst, die im Wesentlichen frei von Kleber ist; oder
wobei die Befestigungsbandanordnung (10) die Verlängerungskomponente (30, 90) umfasst, die mindestens teilweise durch einen Kleber bedeckt ist, wobei optional der Kleber der Verlängerungskomponente (30, 90) mindestens teilweise strukturiert ist, wobei optional der Kleber der Verlängerungskomponente (30, 90) ein druckempfindlicher Kleber ist.

14. Artikel (1) nach Anspruch 12, wobei der Artikel (1) eine Windel ist, wobei die Windel optional eine Höschenwindel ist.

15. Artikel (1) nach einem der Ansprüche 12-14, wobei die Haftungskomponente (40) und die Verlängerungskomponente (30, 90) getrennt gefertigt sind und der Kleber der Haftungskomponente (40) in einem Klebebereich auf der ersten Fläche (48) der Haftungskomponente (40) bereitgestellt ist, um dadurch eine mindestens teilweise Haftung zwischen der Haftungskomponente (40) und der Verlängerungskomponente (30, 90) herzustellen; oder
wobei die Haftungskomponente (40) und die Verlängerungskomponente (30, 90) zusammen als ein Teil oder eine Einheit gefertigt sind und der Kleber der Haftungskomponente (40) auf die erste Fläche (48) der Haftungskomponente (40) aufgetragen ist.

## Revendications

1. Ensemble bande de fixation (10) comprenant :
un composant de fixation (20) définissant une première face (28) pour la fixation sur un article jetable (1) ou un substrat et une seconde face orientée de manière opposée (29) ;
un composant de raccordement (80), ledit composant de raccordement (80) comprenant :
un composant de liaison (40) définissant une première face (48) pour la fixation à un article jetable (1) ou à un substrat et une seconde face orientée de manière opposée (49) ; et
un composant d'extension (30, 90) globalement disposé entre le composant de fixation (20) et le composant de liaison (40) ;
ladite première face (28) du composant de fixation (20) étant au moins partiellement recouverte par un adhésif ;
ladite première face (48) du composant de liaison (40) étant au moins partiellement recouverte par un adhésif ; et
ledit composant d'extension (30, 90) comprenant un bord plié (31) et comprenant en outre une partie sans adhésif (27) entre le bord plié (31) du composant d'extension (30, 90) et le composant de liaison (40).

2. Ensemble bande de fixation (10) de la revendication 1, ledit adhésif du composant de fixation (20) étant prévu dans une zone adhésive sur la première face (28) du composant de fixation (20) pour ainsi lier le composant de fixation (20) au composant d'extension (30, 90), éventuellement ledit composant de fixation (20) comprenant en outre des crochets (70), éventuellement ledit adhésif du composant de fixation (20) étant au moins partiellement structuré, éventuellement ledit adhésif du composant de fixation (20) étant un adhésif sensible à la pression.

3. Ensemble bande de fixation (10) de la revendication 1 ou de la revendication 2, ledit composant de liaison (40) et ledit composant d'extension (30, 90) étant fabriqués séparément et ledit adhésif du composant de liaison (40) étant prévu dans une zone adhésive (44) sur la première face (48) du composant de liaison (40) pour ainsi lier au moins partiellement le composant de liaison (40) au composant d'extension (30, 90) ; ou
ledit composant de liaison (40) et ledit composant d'extension (30, 90) étant fabriqués ensemble d'une seule pièce ou unité et ledit adhésif du composant de liaison (40) étant appliqué sur la première face (48) du composant de liaison (40).

4. Ensemble bande de fixation (10) de l'une quelconque des revendications 1 à 3, ledit adhésif du composant de liaison (40) étant au moins partiellement structuré, éventuellement ledit adhésif du composant de liaison (40) étant un adhésif sensible à la pression, éventuellement ledit bord plié (31) du composant d'extension (30, 90) étant une liaison en U ou une liaison en Y.

5. Ensemble bande de fixation (10) de l'une quelconque des revendications 1 à 4, ledit ensemble de fixation (10) comprenant en outre une tirette (50) fixée au moins partiellement au composant de fixation (20), éventuellement ledit ensemble de fixation (10) comprenant en outre au moins une perforation (60) dans le composant de fixation (20), éventuellement un bord de raccordement (22) du composant de fixation (20) s'étendant au-delà du composant d'extension (30, 90).

6. Ensemble bande de fixation (10) de l'une quelconque des revendications 1 à 5, ledit composant d'extension (30, 90) et ledit composant de liaison (40) s'étendant au-delà du bord distal (21) du composant de fixation (20), éventuellement ledit bord distal (21) du composant de fixation (20) s'étendant au-delà du composant d'extension (30, 90) et du composant de liaison (40), éventuellement, ledit bord distal (21) du composant de fixation (20) s'étendant au-delà du bord d'extension séparé du composant d'extension (30, 90).

7. Ensemble bande de fixation (10) de l'une quelconque des revendications 1 à 6, ledit composant d'extension (30, 90) étant essentiellement exempt d'adhésif ; ou
ledit composant d'extension (30, 90) étant au moins partiellement recouvert d'un adhésif, éventuellement ledit adhésif du composant d'extension (30, 90) étant au moins partiellement structuré, éventuellement ledit adhésif du composant d'extension (30, 90) est un adhésif sensible à la pression.

8. Ensemble bande de fixation (10) de l'une quelconque des revendications 1 à 7, comprenant en outre une couche de séparation (37) sur au moins l'un du composant de liaison (40) et du composant d'extension (30, 90), comprenant éventuellement en outre au moins une perforation éventuelle (60) dans le composant de fixation (20).

9. Ensemble bande de fixation (10) de l'une quelconque des revendications 1 à 8, ledit ensemble bande de fixation (10) étant sous forme de rouleau.

10. Procédé de fabrication de l'ensemble bande de fixation (10) selon l'une quelconque des revendications 1 à 9, le procédé comprenant les étapes de :
fourniture d'un composant de fixation (20) définissant une première face (28) pour la fixation à un article jetable (1) et une seconde face orientée de manière opposée (29) ;
fourniture d'un composant de raccordement (80) comprenant un composant de liaison (40) définissant une première face (48) pour la fixation à un article jetable ou un substrat et une seconde face orientée de manière opposée (49) ; et
fourniture d'un composant d'extension (30, 90) disposé entre le composant de fixation (20) et le composant de liaison (40),
recouvrement au moins partielle de la première face (28) du composant de fixation (20) avec un adhésif ;
recouvrement au moins partielle de la première face (48) du composant de liaison (40) avec un adhésif ; et
fourniture au composant d'extension (30, 90) d'un bord plié (31) et d'une partie sans adhésif entre le bord plié (31) et le composant de liaison (40).

11. Article comprenant un ensemble bande de fixation (10) de l'une quelconque des revendications 1 à 9.

12. Article (1) comprenant un ensemble bande de fixation (10), l'ensemble de bande de fixation (10) comprenant :
un composant de fixation (20) définissant une première face (28) pour la fixation sur un article jetable (1) ou un substrat et une seconde face orientée de manière opposée (29) ; et
un composant de raccordement (80), ledit composant de raccordement (80) comprenant :
un composant de liaison (40) définissant une première face (48) pour la fixation à un article jetable (1) ou à un substrat et une seconde face orientée de manière opposée (49) ; et
un composant d'extension (30, 90) globalement disposé entre le composant de fixation (20) et le composant de liaison (40) ;
ladite première face (28) du composant de fixation (20) étant au moins partiellement recouverte par un adhésif ;
ladite première face (48) du composant de liaison (40) étant au moins partiellement recouverte par un adhésif ; et
ledit composant d'extension (30, 90) comprenant un bord plié (31) et comprenant en outre une partie sans adhésif (27) entre le bord plié (31) du composant d'extension (30, 90) et le composant de liaison (40).

13. Article (1) de la revendication 12, ledit ensemble bande de fixation (10) comprenant le composant d'extension (30, 90) essentiellement exempt d'adhésif ; ou
ledit ensemble bande de fixation (10) comprenant le composant d'extension (30, 90) au moins partiellement recouvert par un adhésif, éventuellement ledit adhésif du composant d'extension (30, 90) étant au moins partiellement structuré, éventuellement ledit adhésif du composant d'extension (30, 90) étant un adhésif sensible à la pression.

14. Article (1) de la revendication 12, ledit article (1) étant une couche, éventuellement ladite couche étant une couche-culotte.

15. Article (1) de l'une quelconque des revendications 12 à 14, ledit composant de liaison (40) et ledit composant d'extension (30, 90) étant fabriqués séparément et ledit adhésif du composant de liaison (40) étant prévu dans une zone adhésive sur la première face (48) du composant de liaison (40) pour lier ainsi au moins partiellement le composant de liaison (40) au composant d'extension (30, 90) ; ou
ledit composant de liaison (40) et ledit composant d'extension (30, 90) étant fabriqués ensemble d'une seule pièce ou unité et ledit adhésif du composant de liaison (40) étant appliqué sur la première face (48) du composant de liaison (40).
